# FASCICULE DE BREVET EUROPEEN

(11) **EP 0 462 016 B1**
(45) Date de publication et mention de la délivrance du brevet: **29.09.1999**
(21) Numéro de dépôt: 91401582.1
(22) Date de dépôt: 14.06.1991
(51) Int. Cl.: C07D 209/08, A61K 31/40

(54) **Procéde de préparation industrielle du 4-chloro 3-sulfamoyl N-(2,3-dihydro 2-méthyl 1H-indol-1-yl) benzamide**
Verfahren zur industriellen Herstellung von 4-Chloro-3-sulfamoyl-N-(2,3-dihydro-2-methyl-1H-indol-1-yl)-Benzamid
Process for the industrial preparation of 4-chloro-3-sulfamoyl-N-(2,3-dihydro-2-methyl-1H-indol-1-yl) benzamide

(30) Priorité: 14.06.1990 FR 9007386
(43) Date de publication de la demande: 18.12.1991
(73) Titulaire: ADIR ET COMPAGNIE, 92415 Courbevoie Cédex (FR)
(72) Inventeur: Cohen, Armand, F-76210 Bolbec (FR)
(74) Mandataire: Reverbori, Marcelle

(56) Documents cités:
- EP-A- 0 054 892
- EP-A- 0 068 239
- FR-A- 2 003 311
- GB-A- 1 095 040
- US-A- 4 564 677
- Methoden der organischen Chemie (Houben-Weyl),X/2,pp296-300
- Zeitschrift für Chemie,Vol.10,pp 68-69

## Description

La présente invention concerne un nouveau procédé de préparation industrielle du 4-chloro 3-sulfamoyl N-(2,3-dihydro 2-méthyl 1H-indol-1-yl) benzamide.

Ce composé, aussi connu sous la DCI indapamide, possède des propriétés pharmacologiques très intéressantes. Il est doué notamment de propriétés antihypertensives et il est utilisé pour le traitement de l'hypertension artérielle essentielle.

Plusieurs méthodes de préparation de ce composé sont déjà connues. Toutefois, les procédés déjà décrits dans la littérature, ne permettent pas toujours d'obtenir l'indapamide avec une pureté satisfaisante ou avec un bon rendement. De plus, certaines étapes de ces procédés peuvent poser des problèmes au niveau industriel.

Le procédé de préparation du 4-chloro 3-sulfamoyl N-(2,3-dihydro 2-méthyl 1H-indol-1-yl) benzamide décrit dans le brevet FR 2.003.311 consiste à obtenir ce composé en faisant réagir le chlorure de 4-chloro 3-sulfamoyl benzoyle sur le 1-amino 2,3-dihydro 2-méthyl indole. Ce dernier composé est préparé selon le procédé de J.B. Wright et R.E. Willette (J. Med. and Pharm. Chem. 5 811 (1962) qui comprend la réduction de l'analogue N-nitrosé. Le rendement des différentes étapes de ce procédé est peu satisfaisant.

Par ailleurs, ce procédé de synthèse conduit à l'obtention de produits secondaires, notamment des dérivés de nitrosamines, qui sont des composés de toxicité potentielle.

Le brevet JP 54.030.159 décrit un procédé de préparation de l'indapamide à partir de 4-chloro 3-sulfamoyl N-(2-méthyl indol-1-yl) benzamide, qui est transformé en indapamide après réduction.

Ce procédé est très proche du précédent et conduit aussi à la formation d'un grand nombre de produits secondaires, et plusieurs purifications sont nécessaires pour obtenir un produit de qualité pharmaceutique.

Un autre procédé de préparation de l'indapamide est décrit dans le brevet EP 54.892. Ce procédé consiste à cycliser la 1-allyl 1-phényl 2-(3-sulfamoyl 4-chlorobenzoyl) hydrazine en présence d'acides de Lewis ou de Bronsted. Cette cyclisation conduit aussi à la formation d'un grand nombre de produits secondaires, et le rendement de ce procédé est peu satisfaisant. A côté de ces documents antérieurs ayant trait essentiellement à la préparation de l'indapamide, on peut citer également : Méthoden der organischen Chemie (Houben-Weyl) vol X/2 pages 269-300 ; Zeitschrift für Chemie, vol 10, pages 68-69 (1970) et le brevet GB-A-1095040, lesquels, pris en combinaison, indiquent que l'on peut effectuer, avec un bon rendement, l'amination direction d'arylamines au moyen de chloramine gazeuse ou générée en solution aqueuse ; sans toutefois suggérer qu'une telle amination soit applicable aux amines hétérocycliques N-alkylées et en particulier au 2,3-dihydro 2-méthyl 1H-indole.

Compte-tenu de l'intérêt thérapeutique de l'indapamide, et de l'absence d'un procédé industriel permettant son obtention avec une pureté satisfaisante, un bon rendement et si possible, à partir de matières premières peu onéreuses et disponibles dans le commerce, des recherches plus approfondies ont été entreprises et ont abouti à la découverte d'un nouveau procédé de préparation du 4-chloro 3-sulfamoyl N-(2,3-dihydro 2-méthyl 1H-indol-1-yl) benzamide. Le stade final de ce procédé est proche de celui décrit dans le brevet FR 2.003.311 mais il présente deux grands avantages : il nécessite moins d'étapes, et la préparation du 1-amino 2,3-dihydro 2-méthyl 1H-indole est réalisée sans formation de l'analogue N-nitrosé. L'application industrielle de ce procédé est donc très avantageuse.

La présente invention a plus particulièrement pour objet un procédé de synthèse industrielle du 4-chloro 3-sulfamoyl N-(2,3-dihydro 2-méthyl 1H-indol-1-yl) benzamide, composé de formule I : 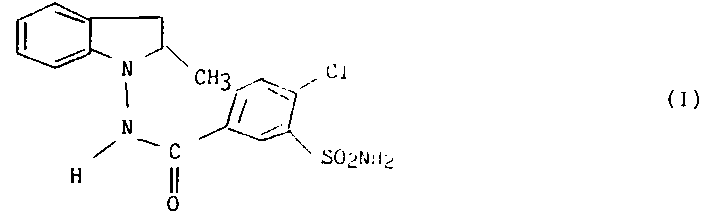 caractérisé en ce que l'on fait réagir une solution d'hydroxyde d'ammonium et de chlorure d'ammonium avec une solution aqueuse d'hypochlorite de sodium à une température comprise entre -10°C et -5°C en milieu alcalin,
et l'on fait réagir la monochloramine ainsi formée avec le 2,3-dihydro 2-méthyl 1H-indole, composé de formule II : 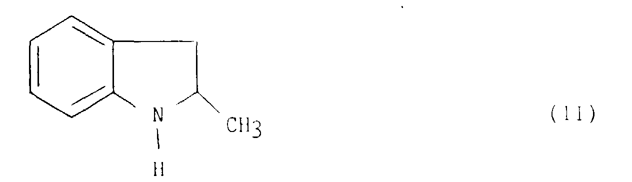 en solution dans un alcool aliphatique ayant 1-5 atomes de carbone, dans un réacteur approprié muni d'un agitateur coaxial à ailettes, à une température comprise entre 30°C et 80°C et en milieu alcalin,
- puis l'on sépare du milieu réactionnel le 1-amino 2,3-dihydro 2-méthyl 1H-indole, composé de formule III : 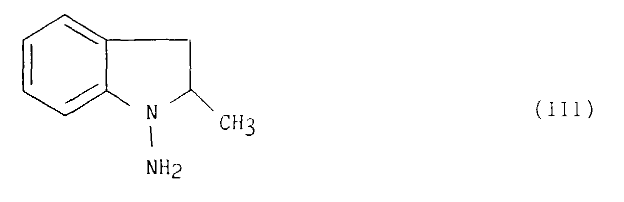
que l'on fait réagir, sous forme de base ou sous forme de sel, en solution dans le tétrahydrofurane ou en milieu alcoolique et en présence d'un accepteur d'acide, avec le chlorure de 4-chloro 3-sulfamoyl benzoyle, composé de formule IV : 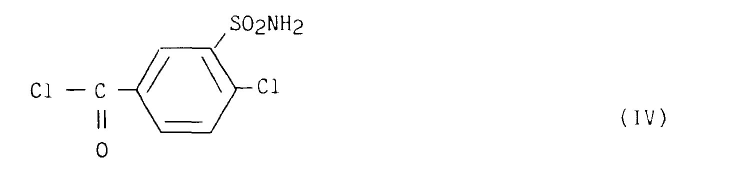 à une température comprise entre 20° et 30°C pour former le composé de formule I.

Le rapport des concentrations molaires : hydroxyde d'ammonium et chlorure d'ammonium sur hypochlorite de sodium est d'environ 2,5 à 3 et le rapport des concentrations molaires : chlorure d'ammonium sur hydroxyde d'ammonium est d'environ 0,50 à 0,80.

La réaction de la monochloramine avec le 2,3-dihydro 2-méthyl 1H-indole s'effectue en continu, à chaud et en présence d'une solution aqueuse d'hydroxyde de sodium.

La concentration de la solution alcoolique du 2,3-dihydro 2-méthyl 1H-indole doit être comprise entre 0,5 et 2 moles/l et de manière préférentielle de 0,5 à 1 mole/l. Comme solvant alcoolique, on peut utiliser de manière préférentielle le méthanol ou l'éthanol. La réaction du 2,3-dihydro 2-méthyl 1H-indole avec la chloramine est réalisée à un pH compris entre 13 et 14. La réaction de la monochloramine avec le 2,3-dihydro 2-méthyl 1H-indole est réalisée à une température comprise entre 30°C - 80°C, et de manière préférentielle entre 40°C et 65°C.

A la fin de la réaction on peut isoler le 1-amino 2,3-dihydro 2-méthyl 1H-indole de la manière suivante :
- Quand la concentration en 2,3-dihydro 2-méthyl 1H-indole, utilisée pour la réaction, est supérieure à 1 mole/l, on peut procéder d'abord à une décantation du milieu réactionnel pour récupérer la phase organique, puis extraire la phase aqueuse par le toluène. Les phases organiques sont ensuite réunies et évaporées à sec pour obtenir le produit attendu.
- Dans le cas où la concentration en 2,3-dihydro 2-méthyl 1H-indole utilisée pour la préparation du 1-amino 2,3-dihydro 2-méthyl 1H-indole est inférieure à 1 mole/l, le milieu réactionnel est extrait directement par le toluène.

Le 1-amino 2,3-dihydro 2-méthyl 1H-indole, ainsi obtenu, peut-être purifié sur colonne (distillation) et ensuite salifié avant d'être utilisé pour la préparation de l'indapamide. De manière préférentielle, on peut utiliser pour la salification, l'acide chlorhydrique ou l'acide méthanesulfonique.

La réaction du 1-amino 2,3-dihydro 2-méthyl 1H-indole avec le chlorure de 4-chloro 3-sulfamoyl benzoyle est effectuée en présence d'un excès de triéthylamine. Comme solvant réactionnel, on peut utiliser le tétrahydrofurane ou un alcool.

Ci-après est donnée à titre non limitatif une description détaillée de la mise en oeuvre du procédé de l'invention.

### EXEMPLE 1

### Préparation du 1-amino 2,3-dihydro 2-méthyl 1H-indole

Une solution d'hypochlorite de sodium titrant 48° chlorométriques et une solution contenant 3,60 moles/l d'ammoniac et 2,38 moles/l de chlorure d'ammonium sont introduites en continu et sous agitation dans un réacteur (R₁) à raison de 10,53 et 8,79 g/min respectivement.

La température au sein du réacteur R₁ est maintenue entre -8° et -10°C et le pH de la réaction est voisin de 10.

A la sortie de R₁, on obtient une solution de monochloramine qui est introduite dans un réacteur cylindrique (R₂) agité énergiquement au moyen d'un agitateur coaxial à ailettes.

Dans le réacteur R₂, est introduite aussi une solution alcoolique du 2,3-dihydro 2-méthyl 1H-indole (C = 1 mole/l - Q totale = 2,415 g) avec un débit de 11,08 g/min et une solution aqueuse d'hydroxyde de sodium à 30 % avec un débit de 7,95 g/min. Cette dernière solution est utilisée pour maintenir le pH du milieu réactionnel à environ 13,5. La température de la réaction est de 60°C.

A la fin de la réaction, après environ 4 heures, le milieu réactionnel est décanté. La phase organique (∼ 800 g) est récupérée et ensuite, la phase aqueuse est extraite une première fois par 750 ml de toluène et une deuxième fois par 300 ml de toluène.

Les phases organiques sont réunies et le toluène éliminé. Le résidu de l'évaporation est ensuite distillé sur colonne pour obtenir le 1-amino 2,3-dihydro 2-méthyl 1H-indole pur.
- Rendement : 85 %
- Point de fusion : 37 - 38°C

Le 1-amino 2,3-dihydro 2-méthyl 1H-indole est salifié avec une quantité adéquate d'acide méthanesulfonique pour obtenir le mésylate du 1-amino 2,3-dihydro 2-méthyl 1H-indole.
- Point de fusion (Kofler) : 165 - 172°C

### EXEMPLE 2

### Préparation du 3-aminosulfonyl 4-chloro N-(2,3-dihydro 2-méthyl 1H-indol-1-yl) benzamide

Dans un réacteur (R₃), purgé à l'azote, on charge 800 ml de tétrahydrofurane et 0,2045 mole de mésylate du 1-amino 2,3-dihydro 2-méthyl 1H-indole. On coule ensuite en 10 minutes 0,4328 mole de triéthylamine. La solution réactionnelle est agitée ensuite à 18 - 20°C pendant 30 minutes. On coule alors goutte à goutte en 1 h 45 min 0,2050 mole de chlorure de 4-chloro 3-sulfamoyl benzoyle en solution dans 400 ml de tétrahydrofurane.

On laisse le milieu réactionnel sous agitation pendant 3 heures à 24 - 30°C, puis on ajoute 0,5 g de Noir CN₁®. On filtre et on concentre le filtrat.

Le résidu de l'évaporation est ensuite recristallisé dans l'isopropanol.
- Rendement : 80,5 %
- Pureté (CLHP) : 99,98 %

## Revendications

1. Procédé de préparation du 4-chloro 3-sulfamoyl N-(2,3-dihydro 2-méthyl 1H-indol-1yl) benzamide, composé de formule I : 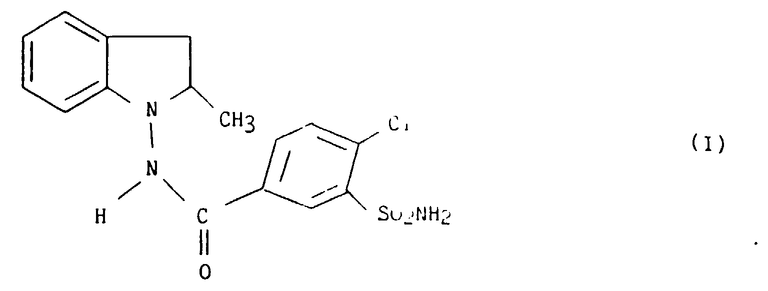 caractérisé en ce que l'on fait réagir une solution d'hydroxyde d'ammonium et de chlorure d'ammonium avec une solution aqueuse d'hypochlorite de sodium à une température comprise entre -10°C et -5°C, en milieu alcalin, et l'on fait réagir la monochloramine ainsi formée avec le 2,3-dihydro 2-méthyl 1H-indole, composé de formule II : 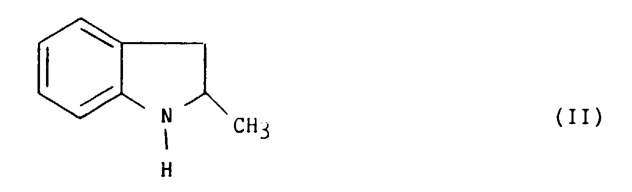 en solution dans un alcool aliphatique ayant 1-5 atomes de carbone, à une température comprise entre 30°C et 80°C et en milieu alcalin,
puis l'on sépare le 1-amino 2,3-dihydro 2-méthyl lH-indole, composé de formule III : 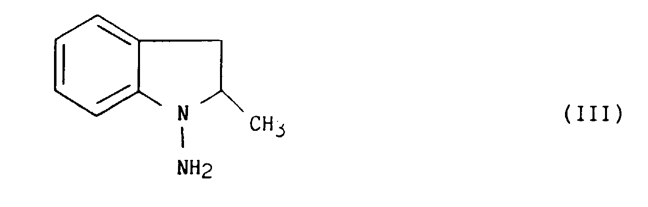
ainsi formé,
que l'on fait réagir sous forme de base ou sous forme de sel, en solution dans le tétrahydrofurane ou en milieu alcoolique et en présence d'un accepteur d'acide avec le chlorure de 4-chloro 3-sulfamoyl benzoyle, composé de formule IV : 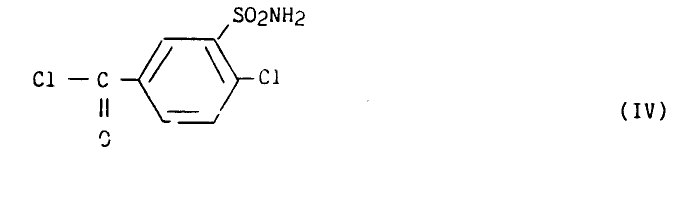 à une température comprise entre 20° et 30°C pour former le composé de formule I.

## Claims

1. Process for the preparation of 4-chloro-3-sulphamoyl-N-(2,3-dihydro-2-methyl-1H-indol-1-yl)benzamide, compound of formula I: 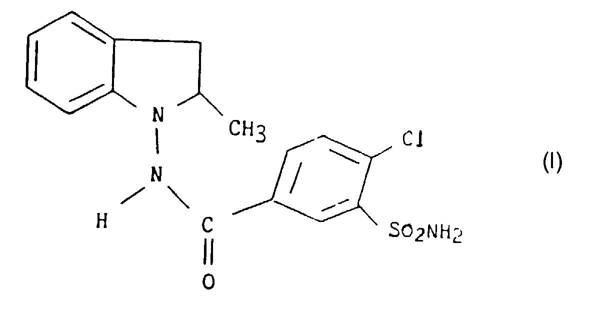 characterised in that a solution of ammonium hydroxide and ammonium chloride is reacted with an aqueous sodium hypochlorite solution at a temperature of from -10°C to -5°C, in an alkaline medium, and the resulting monochloramine is reacted with 2,3-dihydro-2-methyl-1 H-indole, compound of formula II: 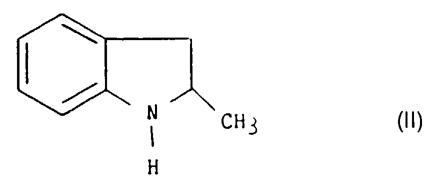 in solution in an aliphatic alcohol having from 1 to 5 carbon atoms, at a temperature of from 30°C to 80°C and in an alkaline medium,
and then the resulting 1-amino-2,3-dihydro-2-methyl-1H-indole, compound of formula III: 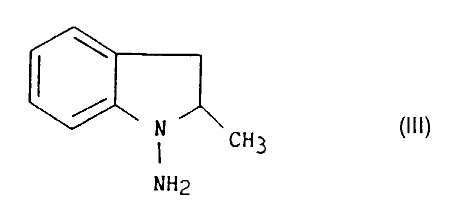 is separated and reacted in the form of a base or in the form of a salt, in solution in tetrahydrofuran or in an alcoholic medium and in the presence of an acid acceptor with 4-chloro-3-sulphamoylbenzoyl chloride, compound of formula IV: 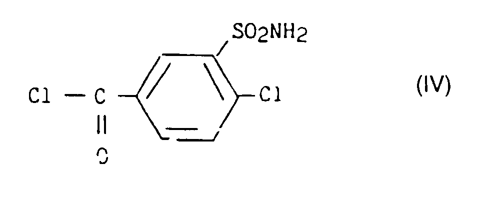 at a temperature of from 20°C to 30°C to form the compound of formula I.

## Patentansprüche

1. Verfahren zur Herstellung von 4-Chlor-3-sulfamoyl-N-(2,3-dihydro-2-methyl-1H-indol-1-yl)-benzamid der Formel I: 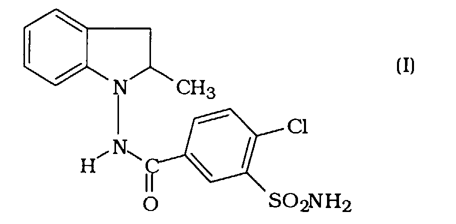 dadurch gekennzeichnet, daß man eine Lösung von Ammoniumhydroxid und Ammoniumchlorid mit einer wäßrigen Lösung von Natriumhypochlorit bei einer Temperatur zwischen -10°C und -5°C in alkalischem Medium umsetzt und das in dieser Weise gebildete Monochloramin mit 2,3-Dihydro-2-methyl-1H-indol der Formel II: 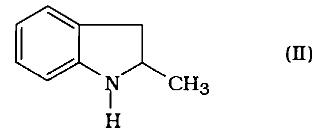 in Lösung in einem aliphatischen Alkohol mit 1 bis 5 Kohlenstoffatomen bei einer Temperatur zwischen 30°C und 80°C in alkalischem Medium umsetzt
und dann das in dieser Weise gebildete 1-Amino-2,3-dihydro-2-methyl-1H-indol der Formel III: 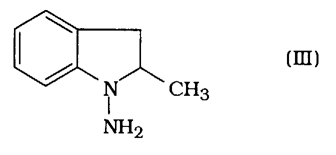 abtrennt und
in Form der Base oder in Form des Salzes in Lösung in Tetrahydrofuran oder in alkoholischem Medium und in Gegenwart eines Säureakzeptors mit 4-Chlor-3-sulfamoyl-benzoylchlorid der Formel IV: 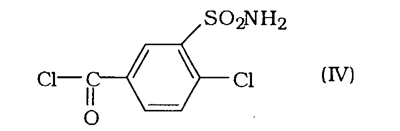 bei einer Temperatur zwischen 20 und 30°C zur Bildung der Verbindung der Formel I umsetzt.
